# EUROPÄISCHE PATENTSCHRIFT

(11) **EP 2 448 663 B1**
(45) Veröffentlichungstag und Bekanntmachung des Hinweises auf die Patenterteilung: **17.04.2013**
(21) Anmeldenummer: 10725034.2
(22) Anmeldetag: 09.06.2010
(51) Int. Cl.: B01J 19/12, C07C 67/08, C07C 69/24, C07C 69/52, C07C 1/32, C07C 15/14, C07C 51/567, C07C 55/02, C07C 231/02, C07C 233/05, C07C 233/09, C07C 233/36, C07D 307/60, H05B 6/80

(54) **VORRICHTUNG ZUR KONTINUIERLICHEN DURCHFÜHRUNG CHEMISCHER REAKTIONEN BEI HOHEN TEMPERATUREN**
DEVICE FOR CONTINUOUSLY CARRYING OUT CHEMICAL REACTIONS AT HIGH TEMPERATURES
DISPOSITIF DESTINÉ À LA RÉALISATION EN CONTINU DE RÉACTIONS CHIMIQUES À HAUTES TEMPÉRATURES

(30) Priorität: 30.06.2009 DE 102009031059
(43) Veröffentlichungstag der Anmeldung: 09.05.2012
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE)
(74) Vertreter: Mikulecky, Klaus
(86) Internationale Anmeldenummer: PCT/EP2010/003443
(87) Internationale Veröffentlichungsnummer: WO 2011/000460

(56) Entgegenhaltungen:
- EP-A2- 1 291 077
- WO-A1-03/041856
- WO-A1-2008/043494
- US-A- 5 387 397
- ERIK ESVELD ET AL: "Pilot Scale Continuous Microwave Dry-Media Reactor. Part 1: Design and Modeling", CHEMICAL ENGINEERING AND TECHNOLOGY, WEINHEIM, DE, Bd. 23, Nr. 3, 1. Januar 2000 (2000-01-01) , Seiten 279-283, XP007916923, ISSN: 0930-7516
- ERIK ESVELD ET AL: "Pilot Scale Continuous Microwave Dry-Media Reactor - Part II: Application to Waxy Esters Production", CHEMICAL ENGINEERING AND TECHNOLOGY, WEINHEIM, DE, Bd. 23, Nr. 5, 1. Januar 2000 (2000-01-01) , Seiten 429-435, XP007916803, ISSN: 0930-7516 in der Anmeldung erwähnt
- JACHUCK R ET AL: "Process intensification: oxidation of benzyl alcohol using a continuous isothermal reactor under microwave irradiation", GREEN CHEMISTRY, ROYAL SOCIETY OF CHEMISTRY, CAMBRIDGE, GB, Bd. 8, 1. Januar 2006 (2006-01-01), Seiten 29-33, XP007916789, ISSN: 1463-9262, DOI: DOI:10.1039/B512732G [gefunden am 2005-11-14]
- GLASNOV TOMA N ET AL: "Microwave-assisted synthesis under continuous-flow conditions", MACROMOLECULAR: RAPID COMMUNICATIONS, WILEY VCH VERLAG, WEINHEIM, DE, Bd. 28, Nr. 4, 1. Januar 2007 (2007-01-01) , Seiten 395-410, XP002529633, ISSN: 1022-1336, DOI: DOI:10.1002/MARC.200600665

## Beschreibung

Die vorliegende Erfindung betrifft eine Vorrichtung zur kontinuierlichen Durchführung chemischer Reaktionen bei hohen Temperaturen und Überdruck unter Heizen durch Mikrowellenbestrahlung im technischen Maßstab.

Chemische Reaktionen lassen sich durch Temperaturerhöhung beschleunigen. Technisch sind der Temperaturerhöhung jedoch durch die dann auftretenden Drücke Grenzen gesetzt. Zumindest in großen Reaktionsgefäßen von mehreren Litern oder mehreren Kubikmetern ist die Durchführung von Reaktionen unter hohen Drücken auf Grund der dann auftretenden Sicherheitsrisiken wenn überhaupt, dann nur mit hohem technischem Aufwand realisierbar. Weiterhin erfordern in klassischen Rührgefäßen, wie beispielsweise in Pfaudler-Kesseln durchgeführte Reaktionen zur Einstellung der Reaktionstemperatur entsprechend höhere Mantel- oder auch Heizelementtemperaturen, um den erforderlichen Wärmeübergang zu gewährleisten. Dadurch kommt es aber an den Heizflächen zu lokalen Überhitzungen und vielfach auch zu unerwünschten Nebenreaktionen oder auch Zersetzungen des Reaktionsgemischs, was zu verminderter Produktqualität und/oder verminderten Ausbeuten führt.

Die durch hohe Drücke bedingten Sicherheitsrisiken lassen sich zwar durch die Verwendung kontinuierlich betriebener Reaktionsrohre reduzieren, doch bestehen auch hier die durch die erforderlichen Temperaturgradienten hervorgerufenen Probleme beim Wärmeübergang auf das Reaktionsmedium. Für schnelle Heizraten werden hohe Manteltemperaturen benötigt, die wiederum zu unerwünschten Nebenreaktionen oder auch Zersetzungen führen können. Moderate Manteltemperaturen dagegen erfordern zur Erreichung der Zieltemperatur lange Verweilzeiten im Reaktionsrohr und damit niedrige Strömungsgeschwindigkeiten und/oder lange Rohre. Während dieses langsamen Aufheizens werden bei vielen Reaktionen ebenfalls unerwünschte Nebenreaktionen beobachtet.

Ein neuerer Ansatz zur chemischen Synthese ist die Durchführung von Reaktionen im Mikrowellenfeld. Diese Reaktionstechnik wird bisher hauptsächlich im Labormaßstab und nur selten im Kleintechnikumsmaßstab angewendet, da bisher keine Vorrichtungen bekannt sind, die die Herstellung von mehr als wenigen kg pro Tag ermöglichen.

WO-90/03840 offenbart ein kontinuierliches Verfahren zur Durchführung verschiedener chemischer Reaktionen in einem kontinuierlichen Labor-Mikrowellenreaktor. Dabei wird das Reaktionsgut mit variablen Fließgeschwindigkeiten von bis zu 1,4 l/h in einem Multimode-Mikrowellenofen unter Drücken von bis zu 12 bar auf Temperaturen von bis zu 190 °C erhitzt. Das Reaktionsprodukt wird im Wesentlichen sofort nach dem Durchlauf durch die Mikrowellenzone abgekühlt. Die erzielten Umsätze zeigen in vielen Fällen jedoch noch Optimierungspotenzial und der Wirkungsgrad dieses Verfahrens bezüglich der Mikrowellenabsorption des Reaktionsguts ist auf Grund der in Multimode-Mikrowellenapplikatoren auf den Applikatorraum mehr oder weniger homogen verteilten und nicht auf die Rohrschlange fokussierten Mikrowellenenergie niedrig. Eine starke Erhöhung der eingestrahlten Mikrowellenleistung würde hier zu unerwünschten Plasma- Entladungen führen. Weiterhin machen die als Hot-Spots bezeichneten, sich zeitlich verändernden räumlichen Inhomogenitäten des Mikrowellenfeldes eine sichere und reproduzierbare Reaktionsführung im großen Maßstab unmöglich.

EP-A-1 291 077 offenbart einen Mikrowellenreaktor, bei dem eine Flüssigkeit in einem Rohr durch einen Mikrowellenhohlleiter quer zur Ausbreitungsrichtung der stehenden elektromagnetischen Welle geführt wird und in dem Moleküle durch Dissoziation und/oder Ionisation mittels Mikrowellenstrahlung aktiviert werden, um anschließend in einen Reaktionsraum mit weiteren Reaktanden zur Reaktion gebracht zu werden. Auf Grund der sehr kleinen Bestrahlungszone ist zum einen die darin behandelbare Substanzmenge äußerst begrenzt und zum anderen die einbringbare Energiemenge gering. Einem Up-scaling dieses Verfahrens durch Vergrößerung des Rohrquerschnitts steht zudem die üblicherweise auf einige Millimeter bis wenige Zentimeter begrenzte Eindringtiefe von Mikrowellen in das Reaktionsgut entgegen.

Esveld et al., Chem. Eng. Technol. 23 (2000), 429 - 435, offenbaren ein kontinuierliches Verfahren zur Herstellung von Wachsestern, bei dem Fettalkohol und Fettsäure lösemittelfrei in Gegenwart von Montmorillonit verestert werden. Auf einem Förderband wird das Reaktionsgemisch durch Mikrowellenstrahlung binnen 5 Minuten auf Reaktionstemperatur erhitzt und anschließend weitere 30 Minuten zur weitgehenden Entfernung des entstehenden Reaktionswassers bei dieser Temperatur gehalten. Dieses in einem offenen System durchgeführte Verfahren ist naturgemäß nur auf hoch siedende Reaktanden (und Reaktionsprodukte) anwendbar.

Üblicherweise wird das Reaktionsgut bei kontinuierlich im Strömungsrohr durchgeführten, mikrowellenunterstützten Reaktionen sofort nach Verlassen der Bestrahlungszone möglichst schnell abgekühlt, z. B. durch adiabatische Expansion gemäß WO-04/054707.

WO-2003/041856 lehrt eine Durchlauf-Heizung und ein Verfahren zur Durchführung chemischer Reaktionen in einem System, umfassend mindestens einen Aufwärmabschnitt mit einer Mikrowellen-Heizeinrichtung und mindestens einem Heizmittel, um die Temperatur des Prozessfluids im Wesentlichen konstant zu halten.

WO-2008/043494 lehrt ein Verfahren zur Herstellung tertiärer Amide von Alkylphenylcarbonsäuren, indem mindestens ein sekundäres Amin mit mindestens einer Alkylphenylcarbonsäure zu einem Ammoniumsalz umgesetzt wird, und dieses Ammoniumsalz nachfolgend unter Mikrowellenbestrahlung weiter zum tertiären Amid umgesetzt wird.

Viele chemische Reaktionen erfordern neben einer schnellen und gezielten Aufheizung zur Einstellung des chemischen Gleichgewichts und damit zur Optimierung der Ausbeute eine gewisse Verweilzeit bei der angestrebten Reaktionstemperatur. Daher wäre zur Einstellung des chemischen Gleichgewichts und damit zur Erzielung möglichst hoher Ausbeuten eine entsprechende Verweilzeit in der Mikrowellenbestrahlungszone wünschenswert, was aber den Durchsatz und damit die Raum-Zeit-Ausbeute mindert.

Aufgabe der Erfindung war demzufolge die Bereitstellung einer Vorrichtung zur kontinuierlichen Durchführung chemischer Reaktionen im technischen Maßstab bei hohen Temperaturen, bei dem das Reaktionsgut möglichst schnell und ohne partielle Überhitzung auf die gewünschte Reaktionstemperatur erhitzt und sodann für einen definierten Zeitraum bei dieser Reaktionstemperatur gehalten und anschließend abgekühlt werden kann. Weiterhin soll die Vorrichtung ein Arbeiten oberhalb des Atmosphärendruckes erlauben, so dass alle Komponenten des Reaktionsgemischs im flüssigen Zustand verbleiben. Die Vorrichtung soll eine hohe Raum-Zeit-Ausbeute, eine hohe energetische Effizienz und darüber hinaus ein sicheres und reproduzierbares Arbeiten ermöglichen.

Überraschenderweise wurde gefunden, dass sich chemische Reaktionen besonders schonend und mit sehr hohen Raum-Zeit-Ausbeuten in einer Vorrichtung durchführen lassen, in der das Reaktionsgut in einem mikrowellentransparenten Rohr durch eine Heizzone geführt wird, in der es mittels Mikrowellen innerhalb kürzester Zeit auf Reaktionstemperatur erhitzt wird und aus der das erhitzte und gegebenenfalls unter Druck stehende Reaktionsgut in eine isotherme Reaktionsstrecke überführt wird, nach deren Verlassen es gegebenenfalls entspannt und abgekühlt wird.

Gegenstand der Erfindung ist eine Vorrichtung zur kontinuierlichen Durchführung chemischer Reaktionen, umfassend einen Mikrowellengenerator, einen Mikrowellenapplikator in dem sich ein mikrowellentransparentes Rohr befindet, und eine isotherme Reaktionsstrecke, die so angeordnet sind, dass das Reaktionsgut im mikrowellentransparenten Rohr, dessen Längsachse sich in der Ausbreitungsrichtung der Mikrowellen befindet, durch einen als Heizzone fungierenden Monomode-Mikrowellenapplikator geführt wird, in dem es mittels Mikrowellen, welche aus dem Mikrowellengenerator in den Mikrowellenapplikator geführt werden, auf Reaktionstemperatur erhitzt wird und in der das erhitzte und gegebenenfalls unter Druck stehende Reaktionsgut direkt nach dem Verlassen der Heizzone in eine sich an die Heizzone anschließende isotherme Reaktionsstrecke überführt und nach Verlassen der isothermen Reaktionsstrecke abgekühlt wird.

Ein weiterer Gegenstand der Erfindung ist ein Verfahren zum kontinuierlichen Durchführen chemischer Reaktionen, bei dem das Reaktionsgut in einem mikrowellentransparenten Rohr, dessen Längsachse sich in der Ausbreitungsrichtung der Mikrowellen befindet, durch eine Heizzone geführt wird, in der es mittels Mikrowellen auf Reaktionstemperatur erhitzt wird und in der das erhitzte und gegebenenfalls unter Druck stehende Reaktionsgut direkt nach dem Verlassen der Heizzone in eine sich an die Heizzone anschließende isotherme Reaktionsstrecke überführt und nach Verlassen der isothermen Reaktionsstrecke abgekühlt wird.

Die erfindungsgemäße Vorrichtung und das erfindungsgemäße Verfahren sind bevorzugt geeignet für solche Reaktionen, die eine gewisse Aktivierungsenergie benötigen. Geeignet sind sie insbesondere für Reaktionen, deren Aktivierungsenergie mindestens 0,01 kJ/mol, bevorzugt mindestens 0,1 kJ/Mol wie beispielsweise 1 bis 100 kJ/mol beträgt. Weiterhin bevorzugt sind die erfindungsgemäße Vorrichtung und das erfindungsgemäße Verfahren für Reaktionen geeignet, die ohne wesentliche exotherme Wärmetönung ablaufen. So sind sie insbesondere für Reaktionen geeignet, deren Wärmetönung ΔH kleiner als -20 kJ/mol und speziell kleiner als -10 kJ/mol wie beispielsweise kleiner als -2 kJ/mol ist. Besonders bevorzugt sind die erfindungsgemäße Vorrichtung und das erfindungsgemäße Verfahren für endotherme Reaktionen, deren Wärmetönung ΔH größer als +0,1 kJ/mol und speziell zwischen +1 kJ/mol und +100 kJ/mol wie beispielsweise zwischen +2 kJ/mol und 70 kJ/mol ist. Beispiele für geeignete chemische Reaktionen sind Veresterungen, Amidierungen, Esterspaltungen, Veretherungen, Acetalisierungen, En-Reaktionen, Diels-Alder Reaktionen, Oxidationen, Reduktionen, Hydrierungen, nukleophile Substitutionen, Additionen, Hydrolysen, Isomerisierungen, Kondensationen, Decarboxylierungen, Eliminierungen und Polymerisationen wie beispielsweise Polykondensationen. Neben den eigentlichen Reaktanden können die Reaktionsgemische auch Hilfsstoffe wie beispielsweise Lösemittel und/oder Katalysatoren zur Beschleunigung der Reaktionen enthalten.

Als Mikrowellen werden elektromagnetische Strahlen mit einer Wellenlänge zwischen 1 cm und 1 m und Frequenzen zwischen 300 MHz und 30 GHz bezeichnet. Dieser Frequenzbereich ist prinzipiell für das erfindungsgemäße Verfahren geeignet. Bevorzugt wird erfindungsgemäß Mikrowellenstrahlung mit den für industrielle, wissenschaftliche, medizinische, häusliche oder ähnliche Anwendungen freigegebenen Frequenzen verwendet, wie beispielsweise Frequenzen von 915 MHz, 2,45 GHz, 5,8 GHz oder 24,12 GHz.

In einer bevorzugten Ausführungsform enthält die erfindungsgemäße Vorrichtung als mikrowellentransparentes Rohr ein druckfestes, chemisch inertes Rohr (Heizrohr), bei dessen Durchströmen das Reaktionsgut der Mikrowellenstrahlung ausgesetzt wird. Für die Mikrowellenbestrahlung werden Monomode-Mikrowellenöfen (bzw. -Mikrowellenapplikatoren) verschiedener Geometrien eingesetzt werden.

Mikrowellengenerator, Mikrowellenapplikator und mikrowellentransparentes Rohr sind so angeordnet, dass das Reaktionsgut im mikrowellentransparenten Rohr durch einen als Heizzone fungierenden Mikrowellenapplikator geführt wird, in dem es mittels Mikrowellen, welche aus dem Mikrowellengenerator in den Mikrowellenapplikator geführt werden, auf Reaktionstemperatur erhitzt wird. Die isotherme Reaktionsstrecke ist so angeordnet, dass das erhitzte und gegebenenfalls unter Druck stehende Reaktionsgut direkt nach dem Verlassen der Heizzone in die sich an die Heizzone anschließende isotherme Reaktionsstrecke übertritt und nach Verlassen der isothermen Reaktionsstrecke abgekühlt wird.

Das Erhitzen des Reaktionsguts erfolgt in einem mikrowellentransparenten, geraden Rohr, dessen Längsachse sich in der Ausbreitungsrichtung der Mikrowellen eines Monomode-Mikrowellenapplikators befindet.

Bevorzugt erfolgt die Bestrahlung des Reaktionsgutes mit Mikrowellen in einem mikrowellentransparenten, geraden Heizrohr, das sich innerhalb eines mit einem Mikrowellengenerator verbundenen Hohlleiters befindet. Bevorzugt fluchtet das Heizrohr axial mit der zentralen Symmetrieachse des Hohlleiters.

Der als Mikrowellenapplikator fungierende Hohlleiter ist bevorzugt als Hohlraumresonator ausgeformt. Weiterhin bevorzugt werden die vom Reaktionsgut im Hohlleiter nicht absorbierten Mikrowellen an seinem Ende reflektiert. Bevorzugt wird die Länge des Hohlraumresonators so dimensioniert, dass sich in ihm eine stehende Welle ausbildet. Durch die Ausformung des Mikrowellenapplikators als Resonator vom Reflexionstyp werden im Applikator eine lokale Erhöhung der elektrischen Feldstärke bei gleicher vom Generator zugeführter Leistung und eine erhöhte Energieausnutzung erzielt.

Der Hohlraumresonator wird bevorzugt im E₀₁ₙ-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen Symmetrieachse ein Maximum und nimmt zur Mantelfläche hin auf den Wert null ab. Diese Feldkonfiguration liegt rotationssymmetrisch um die zentrale Symmetrieachse vor. Durch Verwendung eines Hohlraumresonators mit einer Länge, bei der n eine ganze Zahl ist, wird die Ausbildung einer stehenden Welle ermöglicht. Je nach der gewünschten Strömungsgeschwindigkeit des Reaktionsguts durch das Heizrohr, der gewünschten Temperatur und der dafür benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 3 bis 50 speziell von 4 bis 20 wie beispielsweise drei, vier, fünf, sechs, sieben, acht, neun oder zehn.

Die E₀₁ₙ-Mode des Hohlraumresonators wird in Englischer Sprache auch als TM₀₁ₙ-Mode bezeichnet, siehe beispielsweise K. Lange, K.H. Löcherer, Taschenbuch der Hochfrequenztechnik", Band 2, Seite K21 ff.

Die Einstrahlung der Mikrowellenenergie in den als Mikrowellenapplikator fungierenden Hohlleiter kann über geeignet dimensionierte Löcher oder Schlitze erfolgen. In einer erfindungsgemäß besonders bevorzugten Ausführungsform erfolgt die Bestrahlung des Reaktionsguts mit Mikrowellen in einem Heizrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppelstift durch eine der stirnseitigen Öffnungen in den Hohlraumresonator hinein. Besonders bevorzugt schließt sich das Heizrohr an das Innenleiterrohr des koaxialen Übergangs an und speziell wird es durch dessen Hohlraum hindurch in den Hohlraumresonator geführt. Bevorzugt fluchtet das Heizrohr axial mit einer zentralen Symmetrieachse des Hohlraumresonators. Dazu weist der Hohlraumresonator bevorzugt je eine zentrische Öffnung an zwei gegenüber liegenden Stirnwänden zum Hindurchführen des Heizrohres auf.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Reaktionsgutes mit Mikrowellen in einem mikrowellentransparenten Heizrohr, das sich axialsymmetrisch in einem E₀₁ₙ-Rundhohlleiter mit koaxialem Übergang der Mikrowellen befindet. Bevorzugt wird dabei das Heizrohr durch den Hohlraum eines als Kopplungsantenne fungierenden Innenleiterrohres in den Hohlraumresonator geführt. In einer weiteren bevorzugten Ausführungsform erfolgt die Bestrahlung des Reaktionsgutes mit Mikrowellen in einem mikrowellentransparenten Heizrohr, das durch einen E₀₁ₙ-Hohlraumresonator mit koaxialer Einspeisung der Mikrowellen geführt wird, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich eine stehende Welle mit n = 2 oder mehr Feldmaxima der Mikrowelle ausbildet. In einer weiteren bevorzugten Ausführungsform erfolgt die Bestrahlung des Reaktionsgutes mit Mikrowellen in einem mikrowellentransparenten Heizrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich eine stehende Welle mit n = 2 oder mehr Feldmaxima der Mikrowelle ausbildet.

Mikrowellengeneratoren, wie beispielsweise das Magnetron, das Klystron und das Gyrotron sind dem Fachmann bekannt.

Die zur Mikrowellenbestrahlung eingesetzten Heizrohre sind bevorzugt aus mikrowellentransparentem, hoch schmelzendem Material gefertigt. Besonders bevorzugt werden nichtmetallische Heizrohre eingesetzt. Unter mikrowellentransparent werden hier Werkstoffe verstanden, die selbst möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan δ = ε''/ε' herangezogen. Der dielektrische Verlustfaktor tan δ ist definiert als das Verhältnis aus dielektrischem Verlust ε" und Dielektrizitätskonstante ε'. Beispiele für tan δ-Werte verschiedener Materialien sind beispielsweise in D. Bogdal, Microwave-assisted Organic Synthesis, Elsevier 2005 wiedergegeben. Für erfindungsgemäß geeignete Reaktionsrohre werden Materialien mit bei 2,45 GHz und 25 °C gemessenen tan δ-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Saphir, Zirkonoxid, Siliziumnitrid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Rohrmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Erfindungsgemäße besonders für die Mikrowellenbestrahlung geeignete Heizrohre haben einen Innendurchmesser von einem Millimeter bis ca. 50 cm, insbesondere zwischen 2 mm und 35 cm und speziell zwischen 5 mm und 15 cm wie beispielsweise zwischen 10 mm und 7 cm. Unter Heizrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. Unter der Länge des Heizrohres wird dabei die Strecke des Rohres verstanden, auf der die Mikrowellenbestrahlung erfolgt. In das Heizrohr können Stromstörer und/oder andere Mischelemente eingebaut sein.

Erfindungsgemäß besonders geeignete E₀₁-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung. Bevorzugt hat der E₀₁-Hohlraumresonator einen runden Querschnitt, was auch als E₀₁-Rundhohlleiter bezeichnet wird. Besonders bevorzugt hat er eine zylindrische Form und speziell eine kreiszylindrische Form.

Die Verweilzeit des Reaktionsgutes in der Heizzone hängt von verschiedenen Faktoren wie beispielsweise der Geometrie des Heizrohres, der eingestrahlten Mikrowellenenergie, der spezifischen Mikrowellenabsorption des Reaktionsgutes und der gewünschten Reaktionstemperatur ab. Die Verweilzeit des Reaktionsgutes in der Heizzone beträgt üblicherweise weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten. Die Intensität (Leistung) der Mikrowellenstrahlung wird dabei so eingestellt, dass das Reaktionsgut beim Verlassen der Heizzone die gewünschte Reaktionstemperatur besitzt.

Die für die Durchführung des erfindungsgemäßen Verfahrens in den Hohlraumresonator einzustrahlende Mikrowellenleistung ist insbesondere abhängig von der angestrebten Reaktionstemperatur, aber auch von der Geometrie des Heizrohres und damit des Reaktionsvolumens sowie der Durchflussgeschwindigkeit des Reaktionsguts durch die Heizzone. Die einzustrahlende Mikrowellenleistung liegt üblicherweise zwischen 200 W und mehreren 100 kW und insbesondere zwischen 500 W und 100 kW wie beispielsweise zwischen 1 kW und 70 kW. Sie kann über einen oder mehrere Mikrowellengeneratoren erzeugt werden. Zur Optimierung der Raum-Zeit-Ausbeute wird die Mikrowellenleistung bevorzugt so eingestellt, dass das Reaktionsgemisch in möglichst kurzer Zeit die gewünschte Reaktionstemperatur erreicht, ohne dass es jedoch zu elektrischen Entladungen im Mikrowellenapplikator kommt.

Bevorzugt wird der durch die Mikrowellenbestrahlung bedingte Temperaturanstieg beispielsweise durch Regelung der Mikrowellenintensität und/oder der Durchflussgeschwindigkeit zumindest für Reaktionen der organischen Chemie auf maximal 500 °C begrenzt. Für anorganische Reaktionen sind auch höhere Temperaturen einstellbar. Besonders bewährt hat sich die Durchführung des erfindungsgemäßen Verfahrens bei Temperaturen zwischen 70 und maximal 400 °C insbesondere bei Temperaturen zwischen 120 und maximal 330 °C und speziell zwischen 150 und maximal 300 °C wie beispielsweise bei Temperaturen zwischen 180 und 270 °C.

In der Heizzone können Edukt, Produkt, gegebenenfalls Nebenprodukt und, sofern anwesend, Lösemittel durch die Temperaturerhöhung zu einem Druckaufbau führen. Dieser Überdruck wird bevorzugt erst nach Passieren der Reaktionsstrecke entspannt, wobei die Entspannung zur Verflüchtigung und Abtrennung von überschüssigen Edukt(en), Produkt, Nebenprodukt sowie gegebenenfalls Lösemittel und/oder zur Abkühlung des Reaktionsprodukts genutzt werden kann.

Die Umsetzung des Reaktionsguts beginnt oftmals schon in der Heizzone, ist üblicherweise aber an deren Ende noch nicht im chemischen Gleichgewicht. Nach Erreichen der Reaktionstemperatur wird das Reaktionsgut direkt, das heißt ohne Zwischenkühlung, aus dem Heizrohr in die isotherme Reaktionsstrecke überführt. Bevorzugt ist die Temperaturdifferenz zwischen Verlassen der Heizzone bis zum Eintritt in die isotherme Reaktionsstrecke somit kleiner als ± 30 °C, bevorzugt kleiner ± 20 °C, besonders bevorzugt kleiner ± 10 °C und insbesondere kleiner ± 5 °C. In einer speziellen Ausführungsform entspricht die Temperatur des Reaktionsguts beim Eintritt in die Reaktionsstrecke der Temperatur beim Verlassen der Heizzone. In einer weiteren speziellen Ausführungsform können dem Reaktionsgut vor dem Eintritt in die isotherme Reaktionsstrecke weitere Reaktanden und/oder Hilfsstoffe zugesetzt werden.

Unter der direkten Verbindung zwischen der Heizzone und der isothermen Reaktionsstrecke ist eine Verbindung zu verstehen, die keine aktiven Maßnahmen zum Zuführen und insbesondere zum Abführen von Wärme aufweist.

Als isotherme Reaktionsstrecke kommen alle chemisch inerten Gefäße in Betracht, die ein Verweilen der Reaktionsgemische bei der in der Heizzone eingestellten Temperatur ermöglichen. Unter isothermer Reaktionsstrecke wird verstanden, dass die Temperatur des Reaktionsgutes in der Reaktionsstrecke gegenüber der Eintrittstemperatur auf ± 20 °C. besonders bevorzugt auf ± 10 °C und insbesondere auf ± 5 °C konstant gehalten wird. Somit hat das Reaktionsgut beim Verlassen der Reaktionsstrecke eine Temperatur, die maximal ± 20 °C, besonders bevorzugt ± 10 °C und insbesondere ± 5 °C von der Temperatur beim Eintritt in die Reaktionsstrecke abweicht.

Neben kontinuierlich betriebenen Rührbehältern und Behälterkaskaden sind insbesondere Rohre als isotherme Reaktionsstrecke geeignet. Diese Reaktionsstrecken können aus verschiedenen Materialien wie beispielsweise Metallen, Keramik, Glas, Quarz oder Kunststoffen bestehen mit der Maßgabe, dass diese unter den gewählten Temperatur- und Druckbedingungen mechanisch stabil und chemisch inert sind. Besonders bewährt haben sich dabei thermisch isolierte Gefäße. Die Verweilzeit des Reaktionsguts in der Reaktionsstrecke kann beispielsweise über das Volumen der Reaktionsstrecke eingestellt werden. Bei Verwendung von Rührbehältern und Behälterkaskaden hat es sich gleichermaßen bewährt, die Verweilzeit über den Füllgrad der Behälter einzustellen.

In einer bevorzugten Ausführungsform wird als isotherme Reaktionsstrecke ein Rohr verwendet. Dabei kann es sich um eine Verlängerung des mikrowellentransparenten Heizrohres oder auch ein separates, mit dem Heizrohr in Verbindung stehendes Rohr aus gleichem oder unterschiedlichem Material handeln. Über die Länge des Rohres und/oder seinen Querschnitt lässt sich bei gegebener Flussrate die Verweilzeit des Reaktionsgutes bestimmen. Das als Reaktionsstrecke fungierende Rohr ist im einfachsten Fall thermisch isoliert, so dass die beim Eintritt des Reaktionsgutes in die Reaktionsstrecke herrschende Temperatur in den oben gegebenen Grenzen gehalten wird. Dem Reaktionsgut kann in der Reaktionsstrecke aber auch beispielsweise mittels eines Wärmeträgers bzw. Kühlmediums gezielt Energie zu- oder abgeführt werden. Diese Ausführungsform hat sich insbesondere zum Anfahren der Vorrichtung bzw. des Verfahrens sowie zur Durchführung stärker endothermer bzw. exothermer Reaktionen bewährt. So kann die Reaktionsstrecke beispielsweise als Rohrschlange oder Rohrbündel ausgestaltet sein, die sich in einem Heiz- oder Kühlbad befindet oder in Form eines Doppelmantelrohres mit einem Heiz- oder Kühlmedium beaufschlagt werden. Die Reaktionsstrecke kann sich auch in einem weiteren Mikrowellenapplikator befinden, in dem das Reaktionsgut nochmals mit Mikrowellen behandelt wird. Dabei können sowohl im Monomode- wie auch Multimode arbeitende Applikatoren zum Einsatz kommen.

Die Verweilzeit des Reaktionsgutes in der Reaktionsstrecke ist abhängig von der Reaktionsgeschwindigkeit der durchgeführten Reaktion sowie der Geschwindigkeit eventueller unerwünschter Nebenreaktionen. Im Idealfall wird die Verweilzeit in der Reaktionsstrecke so bemessen, dass der durch die herrschenden Bedingungen definierte thermische Gleichgewichtszustand gerade erreicht wird. Üblicherweise liegt die Verweilzeit zwischen 1 Sekunde und 10 Stunden, bevorzugt zwischen 10 Sekunden und 2 Stunde, besonders bevorzugt zwischen 20 Sekunden und 60 Minuten wie beispielsweise zwischen 30 Sekunden und 30 Minuten.

In einer bevorzugten Ausführungsform wird das Reaktionsgut direkt nach Verlassen der isothermen Reaktionsstrecke möglichst schnell auf Temperaturen unterhalb 120 °C, bevorzugt unterhalb 100 °C und speziell unterhalb 60 °C abgekühlt. Dies kann beispielsweise mittels Wärmetauscher, adiabatischer Expansion oder Verdünnen mit kaltem Lösemittel erfolgen.

Die erfindungsgemäße Vorrichtung ist üblicherweise am Einlass zumindest mit einer Dosierpumpe sowie einem Manometer versehen. Am Übergang zwischen Heizzone und isothermer Reaktionsstrecke befindet sich bevorzugt zumindest eine Temperaturmesseinrichtung. Zur Erhöhung der Sicherheit von Vorrichtung und Verfahren hat es sich bewährt, in den Übergang zwischen Heizzone und isothermer Reaktionsstrecke zudem ein Rückschlagventil zu installieren. Bei längeren isothermen Reaktionsstrecken können diese auch durch weitere Rückschlagventile in mehrere Segmente unterteilt sein. In einer bevorzugten Ausführungsform ist die Reaktionsstrecke mit mindestens einer Druckentlastungsvorrichtung gegen Überdruck gesichert. Am Auslass der isothermen Reaktionsstrecke wird das Reaktionsgut abgekühlt und entspannt. Dazu ist die erfindungsgemäße Vorrichtung üblicherweise zumindest mit einer Druckhaltevorrichtung, einer Temperaturmessung und einer Kühlvorrichtung wie beispielsweise einem Wärmetauscher versehen. Üblicherweise erfolgt die Entspannung des Reaktionsgemischs auf Atmosphärendruck, sie kann aber für anschließende Verfahrensschritte bzw. bei Verwendung spezieller Apparaturen auch auf höhere oder niedrigere Drücke erfolgen. So hat es sich beispielsweise bewährt, das Reaktionsgemisch zur Abtrennung von Lösemittel und/oder unumgesetzten Edukten auf Drücke deutlich unterhalb des Atmosphärendrucks zu entspannen. Die Abkühlung kann in Abhängigkeit von den Eigenschaften der umgesetzten Produkte und der vorgesehenen weiteren Verfahrensschritte vor oder auch nach Druckabsenkung oder bei einem dazwischen liegenden Druck erfolgen.

Die Herstellung der Reaktionsgemische kann kontinuierlich, diskontinuierlich oder auch in semi-Batch-Prozessen durchgeführt werden. So kann die Herstellung des Reaktionsgemischs in einem vorgelagerten (semi)-Batch Prozess durchgeführt werden wie beispielsweise in einem Rührbehälter. Das Reaktionsgemisch wird bevorzugt in-situ erzeugt und nicht isoliert. In einer bevorzugten Ausführungsform werden die Edukte, unabhängig voneinander gegebenenfalls mit Lösemittel verdünnt, erst kurz vor dem Eintritt in das Reaktionsrohr vermischt. So hat es sich besonders bewährt, die Komponenten des Reaktionsgemischs in einer Mischstrecke zusammen zu bringen, aus der sie, gegebenenfalls nach Zwischenkühlung, in die Heizzone gefördert werden. Weiterhin bevorzugt werden die Edukte dem erfindungsgemäßen Verfahren in flüssiger Form zugeführt. Dazu können höher schmelzende und/oder höher viskose Edukte beispielsweise in geschmolzenem Zustand und/oder mit Lösemittel versetzt beispielsweise als Lösung, Dispersion oder Emulsion eingesetzt werden. Ein Katalysator kann, sofern eingesetzt, einem der Edukte oder auch der Eduktmischung vor dem Eintritt in das Heizrohr zugesetzt werden. Auch heterogene Systeme können nach dem erfindungsgemäßen Verfahren umgesetzt werden, wobei entsprechende technische Vorrichtungen zum Fördern des Reaktionsgutes erforderlich sind.

Zur Vermeidung von Nebenreaktionen und zur Herstellung von möglichst reinen Produkten hat es sich bewährt, Edukte und Produkte in Gegenwart eines inerten Schutzgases wie beispielsweise Stickstoff, Argon oder Helium zu handhaben.

Das Reaktionsgut kann in das Reaktionsrohr entweder an dem durch das Innenleiterrohr geführte Ende eingespeist werden als auch an dem entgegen gesetzten Ende. Das Reaktionsgemisch kann folglich parallel oder antiparallel zur Ausbreitungsrichtung der Mikrowellen durch den Mikrowellenapplikator geführt werden.

Zur Durchführung des erfindungsgemäßen Verfahrens werden durch Wahl von Rohrquerschnitt, Länge der Heizzone (hierunter wird die Strecke des Rohres verstanden, in dem das Reaktionsgut Mikrowellenstrahlung ausgesetzt ist), Fließgeschwindigkeit, Geometrie des Hohlraumresonators sowie der eingestrahlten Mikrowellenleistung die Reaktionsbedingungen bevorzugt so eingestellt, dass die gewünschte Reaktionstemperatur schnellstmöglich erreicht wird. Die Regelung der für einzelne chemische Reaktionen gewünschten Reaktionsbedingungen erfolgt bevorzugt durch Steuerung der am Ende der Heizzone erreichten Temperatur des Reaktionsgemischs über die eingestrahlte Mikrowellenleistung und/oder über die Durchflussgeschwindigkeit des Reaktionsgemischs durch die Heizzone. Der Druck wird dabei über das Entspannungsventil (Druckhaltevorrichtung) am Ende der Reaktionsstrecke so hoch eingestellt, dass das Reaktionsgemisch inklusive anfallender Produkte und Nebenprodukte nicht siedet.

Bevorzugt wird das Verfahren bei Drücken zwischen 1 bar (Atmosphärendruck) und 500 bar und besonders bevorzugt zwischen 1,5 und 200 bar, insbesondere zwischen 3 bar und 150 bar und speziell zwischen 10 bar und 100 bar wie beispielsweise zwischen 15 bar und 50 bar durchgeführt. Besonders bewährt hat sich das Arbeiten unter erhöhtem Druck, wobei oberhalb der Siedetemperatur (bei Normaldruck) der Edukte, Produkte, des gegebenenfalls anwesenden Lösemittels und/oder der während der Reaktion gebildeten Produkte gearbeitet wird. Besonders bevorzugt wird der Druck so hoch eingestellt, dass das Reaktionsgemisch während der Mikrowellenbestrahlung im flüssigen Zustand verbleibt und nicht siedet.

Die erfindungsgemäße Vorrichtung und das erfindungsgemäße Verfahren erlauben eine sehr schnelle, energiesparende und kostengünstige Durchführung chemischer Reaktionen in hohen Ausbeuten in großtechnischen Mengen. Die Vorteile des erfindungsgemäßen Verfahrens liegen dabei insbesondere in einer sehr schnellen und trotzdem gezielten Erhitzung des Reaktionsguts mittels Mikrowellen auf die angestrebte Reaktionstemperatur, ohne dass es zu wesentlichen Überschreitungen der durchschnittlichen Temperatur des Reaktionsgutes zum Beispiel an der Gefäßwand kommt. Dies ist besonders ausgeprägt bei Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators und insbesondere innerhalb eines E₀₁-Hohlraumresonators beispielsweise mit koaxialem Übergang der Mikrowellen befindet. Mit der erfindungsgemäßen Vorrichtung ist es im Gegensatz zu konventionellen Heiztechniken mit Wärmeübertragung mittels eines Temperaturgradienten möglich, das Reaktionsgemisch bis annähernd an die Zersetzungstemperatur der temperaturempfindlichsten Komponente zu erhitzen und anschließend bei dieser Temperatur bis zur Einstellung des für diese Bedingungen herrschenden Gleichgewichtszustand zu halten.

In der isothermen Reaktionsstrecke kann die eigentliche Reaktion bzw. die Vervollständigung der Reaktion ohne weitere externe thermische Belastung des Reaktionsgutes stattfinden. Dabei erlaubt die erfindungsgemäße Vorrichtung die Durchführung von Reaktionen auch bei sehr hohen Drücken und/oder Temperaturen.

In der erfindungsgemäßen Vorrichtung sowie bei dem sie nutzenden Verfahren wird ein sehr hoher Wirkungsgrad bei der Ausnutzung der in den Hohlraumresonator eingestrahlten Mikrowellenenergie erzielt wird, der üblicherweise über 50 %, oftmals über 80 %, teilweise über 90 % und in speziellen Fällen über 95 % wie beispielsweise über 98 % der eingestrahlten Mikrowellenleistung liegt und somit ökonomische wie auch ökologische Vorteile gegenüber konventionellen Herstellverfahren wie auch gegenüber Mikrowellenverfahren des Standes der Technik bietet.

Erfindungsgemäße Vorrichtung und Verfahren erlauben zudem durch die kontinuierliche Mikrowellenbestrahlung nur kleiner Mengen an Reaktionsgut eine kontrollierte, sichere und reproduzierbare Reaktionsführung. Insbesondere bei Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators befindet, wird das Reaktionsgut während der Mikrowellenbestrahlung parallel zur Ausbreitungsrichtung der Mikrowellen bewegt. So werden bekannte Überhitzungsphänomene durch unkontrollierbare Feldverteilungen, die zu lokalen Überhitzungen durch wechselnde Intensitäten des Mikrowellenfeldes beispielsweise in Wellenbergen und Knotenpunkten führen, durch die Fließbewegung des Reaktionsgutes ausgeglichen. Die genannten Vorteile erlauben es auch, mit hohen Mikrowellenleistungen von beispielsweise mehr als 10 kW oder mehr als 100 kW zu arbeiten und somit in Kombination mit einer nur kurzen Verweilzeit im Heizrohr große Produktionsmengen von 100 und mehr Tonnen pro Jahr in einer Anlage zu bewerkstelligen. Durch die isotherme Reaktionsstrecke wird eine Optimierung der Raum-Zeit-Ausbeute durch nachgelagerte Einstellung des chemischen Gleichgewichts erzielt, da keine Erhöhung der Verweilzeit in der Bestrahlungszone durch Absenkung der Fließgeschwindigkeit erforderlich ist. Damit ist andererseits ein gegenüber der gleichen Vorrichtung ohne Reaktionsstrecke erhöhter Durchsatz in der Heizzone möglich, was die Wirtschaftlichkeit eines solchen mikrowellenunterstützten Verfahrens weiter verbessert.

Dabei war es überraschend, dass trotz der im kontinuierlich durchflossenen Strömungsrohr nur sehr kurzen Verweildauer des Reaktionsgutes im Mikrowellenfeld eine deutliche Erhöhung des Umsatzes der Reaktanden durch die Verwendung einer zusätzlichen Reaktionsstrecke erreicht werden konnte, ohne Bildung nennenswerter Mengen an Nebenprodukten. Bei einer entsprechenden Umsetzung des Reaktionsgutes in einem Strömungsrohr gleicher Dimensionierung unter thermischer Mantelheizung werden zur Erzielung geeigneter Reaktionstemperaturen extrem hohe Wandtemperaturen benötigt, die oftmals zur Bildung gefärbter Spezies führten. Weiterhin überraschend war, dass sich die genannten Umsätze bei Kondensationsreaktionen wie Amidierungen und Veresterungen unter diesen Reaktionsbedingungen ohne Abtrennung des bei der Kondensation gebildeten Reaktionswassers erzielen lassen. Des Weiteren besitzen die nach dem erfindungsgemäßen Verfahren hergestellten Produkte typischerweise sehr niedrige Metallgehalte ohne dass es einer weiteren Aufarbeitung der Rohprodukte bedarf. So liegen die Metallgehalte der nach dem erfindungsgemäßen Verfahren hergestellten Produkte bezogen auf Eisen als Hauptelement üblicherweise unterhalb 25 ppm bevorzugt unterhalb 15 ppm, speziell unterhalb 10 ppm wie beispielsweise zwischen 0,01 und 5 ppm Eisen.

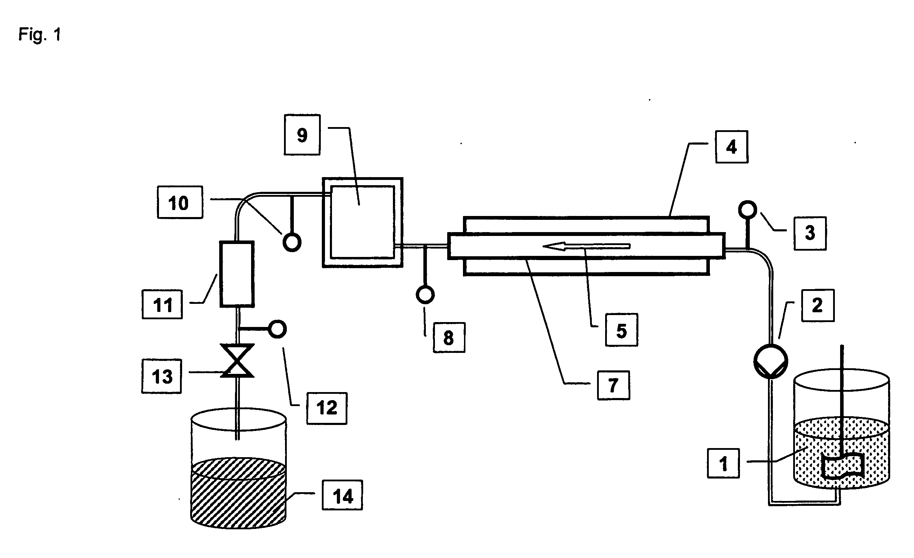

Figur 1 zeigt ein Beispiel für eine erfindungsgemäße Vorrichtung. Sie umfasst eine gerührte Eduktvorlage (1), die über eine Förderleitung mit Förderpumpe (2) das Reaktionsgut bereitstellt. Vor dem Eintritt des Reaktionsguts in das aus mikrowellentransparentem Material gefertigte Heizrohr (7) werden Temperatur und Druck des Reaktionsguts an einer Messstelle (3) bestimmt. Das Heizrohr (7) wird vom Reaktionsgut in der angegebenen Richtung (5) durchströmt. Das Heizrohr befindet sich in einem Mikrowellenapplikator (4). Am Ende der Heizzone befindet sich eine Messstelle für Temperatur und gegebenenfalls Druck (8). Direkt nach Verlassen des Heizrohres (4) wird das Reaktionsgut in die isotherme Reaktionsstrecke (9) überführt. Am Ausgang der isothermen Reaktionsstrecke (9) befindet sich eine Messstelle für Temperatur (10). Hinter der isothermen Reaktionsstrecke ist ein Kühler (11) angebracht, gefolgt von einer Messstelle für Druck und Temperatur (12). Nach Durchlaufen des Kühlers tritt das Produkt durch ein Entspannungsventil (13) in die Produktvorlage (14).

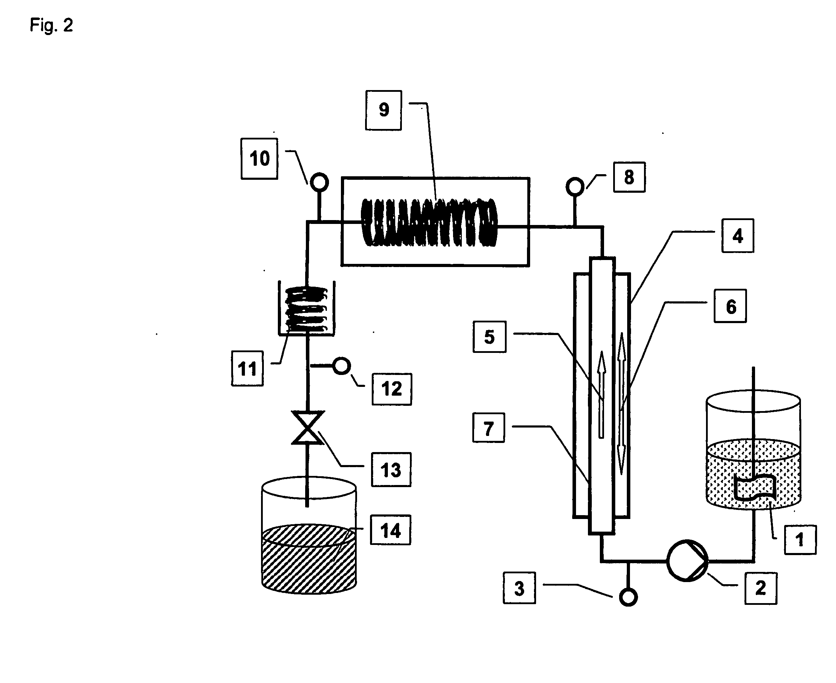

Figur 2 zeigt ein Beispiel für eine weitere erfindungsgemäße Vorrichtung, in der als Mikrowellenofen (4) ein Monomode-Applikator dient, in dem die Ausbreitungsrichtung der Mikrowellen (6) parallel oder antiparallel zur Fließrichtung des Reaktionsgutes (5) ist. Die isotherme Reaktionsstrecke (9) und der Kühler (11) sind hier als Rohrschlangen ausgebildet.

### Beispiele

Die Bestrahlungen der Reaktionsgemische mit Mikrowellen erfolgten in einer Apparatur, die als Heizrohr ein Keramikrohr (60 x 1 cm) enthielt, das sich axialsymmetrisch in einem zylindrischen Hohlraumresonator (60 x 10 cm) befand. An einer der Stirnseiten des Hohlraumresonators verlief dieses Heizrohr durch den Hohlraum eines als Kopplungsantenne fungierenden Innenleiterrohres. Das von einem Magnetron erzeugte Mikrowellenfeld mit einer Frequenz von 2,45 GHz wurde mittels der Kopplungsantenne in den Hohlraumresonator eingekoppelt (E₀₁-Hohlraumapplikator; Monomode), in dem sich eine stehende Welle ausbildete. Die erhitzten Reaktionsgemische wurden unmittelbar anschließend durch ein thermisch isoliertes Edelstahlrohr (3,0 m x 1 cm, sofern nicht anders angegeben) gefördert. Nach Verlassen dieses Reaktionsrohres wurden die Reaktionsgemische auf Atmosphärendruck entspannt und sofort mittels eines Intensivwärmetauschers auf ca. 60 °C abgekühlt.

Die Mikrowellenleistung wurde über die Versuchsdauer jeweils in der Art eingestellt, dass die gewünschte Temperatur des Reaktionsgutes am Ende der Heizzone konstant gehalten wurde. Die in den Versuchsbeschreibungen genannten Mikrowellenleistungen repräsentieren daher den zeitlichen Mittelwert der eingestrahlten Mikrowellenleistung. Die Temperaturmessung des Reaktionsgemischs wurde direkt nach Verlassen der Heizzone (etwa 15 cm Strecke in einer isolierten Edelstahlkapillare, ∅ 1 cm) sowie nach Verlassen der Reaktionsstrecke mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurückgespiegelte Mikrowellenenergie wurde mit Hilfe eines Prismensystems (Zirkulator) in ein Wasser enthaltendes Gefäß geleitet. Aus der Differenz zwischen eingestrahlter Energie und Aufheizung dieser Wasserlast wurde die in das Reaktionsgut eingetragene Mikrowellenenergie berechnet.

Mittels einer Hochdruckpumpe und eines geeigneten Druckentlastungsventils wurde die Reaktionsmischung in der Apparatur unter einen solchen Arbeitsdruck gesetzt, der ausreichte, um alle Edukte und Produkte bzw. Kondensationsprodukte stets im flüssigen Zustand zu halten. Die Reaktionsgemische wurden mit einer konstanten Flussrate durch die Vorrichtung gepumpt und die Verweilzeit in Heizzone und Reaktionsstrecke durch Modifizierung der Strömungsgeschwindigkeit eingestellt.

Die Analytik der Produkte erfolgte mittels ¹H-NMR-Spektroskopie bei 500 MHz in CDCl₃.

### Beispiel 1: Herstellung von N-(3-(N,N-Dimethylamino)propyl)laurylamid

In einem 10 l Büchi-Rührautoklaven mit Rührer, Innenthermometer und Druckausgleich wurden 3,4 kg Laurinsäure (17 mol) vorgelegt, auf 60 °C erwärmt und vorsichtig unter leichter Kühlung mit 2,6 kg N,N-Dimethylaminopropylamin (25,5 mol) versetzt.

Das so erhaltene Gemisch wurde bei einem Arbeitsdruck von 35 bar kontinuierlich mit 10,0 l/h durch die Apparatur gepumpt und dabei in der Heizzone einer Mikrowellenleistung von 4,8 kW ausgesetzt, von denen 94 % vom Reaktionsgut absorbiert wurden. Die Verweilzeit des Reaktionsgemischs in der Heizzone betrug ca. 17 Sekunden, die Verweilzeit in der Reaktionsstrecke ca. 85 Sekunden. Am Ende der Heizzone hatte das Reaktionsgemisch eine Temperatur von 296 °C, nach Verlassen der Reaktionsstrecke eine Temperatur von 292 °C.

Es wurde ein Umsatz der Fettsäure von 97 % d. Th. erreicht. Das Reaktionsprodukt war leicht gelblich gefärbt, der Eisengehalt des Produktes lag unter 5 ppm. Nach destillativer Abtrennung von Reaktionswasser und überschüssigem N,N-Dimethylaminopropylamin wurden 4,6 kg N-(3-(N,N-Dimethylamino)propyl)laurylamid mit einer für die direkte weitere Verwendung (Quaternisierung) ausreichenden Reinheit erhalten.

### Beispiel 2: Herstellung von N,N-Diethylcocosfettsäureamid

In einem 10 l Büchi-Rührautoklaven mit Gaseinleitungsrohr, Rührer, Innenthermometer und Druckausgleich wurden 4,2 kg Cocosfett (5,5 mol / Molekulargewicht 764 g/mol) vorgelegt und auf 45 °C erwärmt. Bei dieser Temperatur wurden langsam 2,0 kg Diethylamin (27 mol) sowie 100 g Natriumethylat als Katalysator zugegeben und unter Rühren homogenisiert.

Das so erhaltene Reaktionsgemisch wurde bei-einem Arbeitsdruck von 32 bar kontinuierlich mit 5,5 l/h durch die Apparatur gepumpt und in der Heizzone einer Mikrowellenleistung von 2,7 kW ausgesetzt, von denen 90 % vom Reaktionsgut absorbiert wurden. Die Verweilzeit des Reaktionsgemischs in der Heizzone betrug ca. 31 Sekunden, die Verweilzeit in der Reaktionsstrecke ca. 155 Sekunden. Am Ende der Heizzone hatte das Reaktionsgemisch eine Temperatur von 260 °C, nach Verlassen der Reaktionsstrecke eine Temperatur von 258 °C.

Das Reaktionsprodukt war leicht gelblich gefärbt. Nach destillativer Abtrennung von überschüssigem Diethylamin, Neutralisation des Katalysators mit verdünnter Essigsäure und Abtrennung der entstandenen Glycerin/Wasser Phase wurden 4,66 kg N,N-Diethylcocosfettsäureamid mit einer Reinheit von > 97 % erhalten.

### Beispiel 3: Herstellung von Rapsölsäuremethylester

In einem 10 I Büchi-Rührautoklaven mit Rührer, Innenthermometer und Druckausgleich wurden 3,1 kg Rapsölfettsäure (10 mol, MW 309 g/mol) vorgelegt und mit 2,58 kg Methanol (80 mol) sowie 0,075 kg Methansulfonsäure versetzt. Das so erhaltene Gemisch wurde bei einem Arbeitsdruck von 35 bar kontinuierlich mit 10,0 l/h durch die Apparatur gepumpt und in der Heizzone einer Mikrowellenleistung von 2,6 kW ausgesetzt, von denen 92 % vom Reaktionsgut absorbiert wurden. Die Verweilzeit des Reaktionsgemischs in der Heizzone betrug ca. 17 Sekunden, die Verweilzeit in der Reaktionsstrecke ca. 86 Sekunden. Am Ende der Heizzone hatte das Reaktionsgemisch eine Temperatur von 251 °C, nach Verlassen der Reaktionsstrecke eine Temperatur von 245 °C.

Es wurde ein Umsatz der Fettsäure von 97 % d. Th. erreicht. Das Reaktionsprodukt war leicht gelblich gefärbt, sein Eisengehalt lag unter 5 ppm. Nach Neutralisation des Katalysators mit Hydrogencarbonat-Lösung, destillativer Abtrennung des überschüssigen Methanols und anschließendem Auswaschen wasserlöslicher Salze wurden 3,1 kg Rapsölmethylester mit einer Restsäurezahl von 0,2 mg KOH/g erhalten.

### Beispiel 4: Suzuki-Kupplung

In einem 1 Liter Dreihalskolben mit Stickstoffinertisierung wurden 500 ml Ethanol vorgelegt und darin 50 g Tetrakis(triphenylphosphine)palladium(0) (44 mmol) unter intensivem Rühren fein suspendiert. In einem mit Stickstoff inertisierten 10 I Büchi-Rührautoklaven mit Rührer, Innenthermometer und Druckausgleich wurden 2 Liter eines Ethanol/Wasser/Dimethylformamid Gemisches vorgelegt und darin 376 g 4-Bromtoluol (2,2 mol) und 244 g (2,0 mol) Phenylboronsäure gelöst. Anschließend wurde die Katalysator-Suspension unter Stickstoff-Inertisierung langsam in den Rührautoklaven gegeben und homogen eingerührt. Die so erhaltene, leicht pumpbare Suspension wurde bei einem Arbeitsdruck von 30 bar kontinuierlich mit 1,5 l/h durch die Apparatur gepumpt und in der Heizzone einer Mikrowellenleistung von 1,2 kW ausgesetzt, von denen 92 % vom Reaktionsgut absorbiert wurden. Die Verweilzeit des Reaktionsgemischs in der Heizzone betrug ca.113 Sekunden, die Verweilzeit in der Reaktionsstrecke ca.10 Minuten. Am Ende der Heizzone hatte das Reaktionsgemisch eine Temperatur von 255 °C, nach Verlassen der Reaktionsstrecke eine Temperatur von 251 °C.

Es wurde eine mittels ¹H-NMR bestimmte Ausbeute (bezogen auf die im Unterschuss eingesetzte Phenylboronsäure) von 72 % d. Th. erreicht, der Eisengehalt des Produktes betrug < 5 ppm. Durch Filtration wurden der Katalysator und unlösliche Nebenprodukte vom Rohprodukt abgetrennt und anschließend das Filtrat destillativ aufgearbeitet. Nach erfolgter Aufarbeitung wurden 256 g 4-Methylbiphenyl mit einer Reinheit von >98 % erhalten.

### Beispiel 5: Herstellung von Poly(isobutenyl)bernsteinsäureanhydrid

In einem 10 I Büchl-Rührautoklaven mit Rührer, Innenthermometer und Druckausgleich wurden 4,0 kg Poly(isobutylen) (Glissopal^{®} 1000, BASF AG, mit Molgewicht 1000, Gehalt an alpha-ständigen Doppelbindungen: 80 %; 4,0 mol) vorgelegt, mit 431 g Maleinsäureanhydrid (4,4 mol) versetzt und unter Rühren auf etwa 70 °C erwärmt.

Die so erhaltene niederviskose Emulsion wurde bei einem Arbeitsdruck von 30 bar kontinuierlich mit 2,0 l/h durch die Apparatur gepumpt und in der Heizzone einer Mikrowellenleistung von 1,8 kW ausgesetzt, von denen 90 % vom Reaktionsgut absorbiert wurden. Die Verweilzeit des Reaktionsgemischs in der Heizzone betrug ca. 85 Sekunden. Als Reaktionsstrecke wurde hier mit einem Rohr mit einem Durchmesser von 2 cm und einer Länge von 10 Metern gearbeitet, so dass eine Verweilzeit in der Reaktionsstrecke von ca. 94 Minuten erreicht wurde. Am Ende der Heizzone hatte das Reaktionsgemisch eine Temperatur von 240 °C, nach Verlassen der Reaktionsstrecke eine Temperatur von 235 °C.

Es wurde ein Umsatz von 82 % des eingesetzten Poly(isobutylens) erzielt. Der Eisengehalt des Produktes betrug < 5 ppm. Eine visuelle Untersuchung der Innenwände von Heizzone und Reaktionszone nach Abschluss des Versuchs ließ in beiden Apparaturteilen keine auf Verkokungen oder Zersetzungen hinweisenden Ablagerungen, wie sie bei konventionell thermisch durchgeführten Reaktionen regelmäßig auftreten, erkennen.

### Beispiele 1V bis 5V: Vergleichsversuche ohne Verwendung einer Reaktionsstrecke

In diesen Versuchen wurden die Versuche 1 bis 5 nur mit der oben beschriebenen Heizzone, das heißt ohne Verwendung eines Reaktionsrohres wiederholt. Die entsprechenden Versuchsparameter sind in der Tabelle 1 zusammengefasst. Die angegebenen Temperaturen beziehen sich auf die beim Verlassen der Heizzone gemessenen Werte.

| Versuch | Durchsatz | µW-Leistung | Verweilzeit in Heizzone | Temperatur nach Heizzone | Umsatz bzw. Ausbeute |
|---|---|---|---|---|---|
| | [l/h] | [kW] | [Sek.] | [°C} | |
| 1 | 10,0 | 4,8 | 17 | 296 | 97 % |
| 1V | 5,6 | 3,1 | 30 | 280 | 93 % |
| 2 | 5,5 | 2,7 | 31 | 260 | 4,66 kg |
| 2V | 4,5 | 2,2 | 38 | 265 | 3,70 kg |
| 3 | 10,0 | 2,6 | 17 | 251 | 97 % |
| 3V | 7,5 | 3,0 | 23 | 279 | 91 % |
| 4 | 1,5 | 1,2 | 113 | 255 | 72 % |
| 4V | 1,0 | 1,0 | 170 | 267 | 42 % |
| 5 | 2,0 | 1,8 | 85 | 240 | 82 % |
| 5V | 1,0 | 1,2 | 170 | 245 | 26 % |

## Patentansprüche

1. Vorrichtung zur kontinuierlichen Durchführung chemischer Reaktionen, umfassend einen Mikrowellengenerator, einen Mikrowellenapplikator in dem sich ein mikrowellentransparentes Rohr befindet, und eine isotherme Reaktionsstrecke, die so angeordnet sind, dass das Reaktionsgut im mikrowellentransparenten Rohr, dessen Längsachse sich in der Ausbreitungsrichtung der Mikrowelle befindet, durch einen als Heizzone fungierenden Monomode-Mikrowellenapplikator geführt wird, in dem es mittels Mikrowellen, welche aus dem Mikrowellengenerator in den Mikrowellenapplikator geführt werden, auf Reaktionstemperatur erhitzt wird und in der das erhitzte und gegebenenfalls unter Druck stehende Reaktionsgut direkt nach dem Verlassen der Heizzone in eine sich an die Heizzone anschließende isotherme Reaktionsstrecke überführt und nach Verlassen der isothermen Reaktionsstrecke abgekühlt wird.

2. Vorrichtung nach Anspruch 1, bei dem sich das mikrowellentransparente Rohr innerhalb eines mit einem Mikrowellengenerator über Wellenleiter verbundenen Hohlleiters befindet.

3. Vorrichtung nach Anspruch 1 oder 2, bei dem der Mikrowellenapplikator als Hohlraumresonator ausgestaltet ist.

4. Vorrichtung nach einem oder mehreren der Ansprüche 1 bis 3, bei dem der Mikrowellenapplikator als Hohlraumresonator vom Reflexionstyp ausgestaltet ist.

5. Vorrichtung nach einem oder mehreren der Ansprüche 1 bis 4, bei dem das mikrowellentranspartente Rohr axial mit einer zentralen Symmetrieachse des Hohlleiters fluchtet.

6. Vorrichtung nach einem oder mehreren der Ansprüche 1 bis 5, bei dem der Hohlraumresonator einen koaxialen Übergang der Mikrowellen aufweist.

7. Vorrichtung nach einem oder mehreren der Ansprüche 1 bis 6, bei dem der Hohlraumresonator im E₀₁ₙ-Mode betrieben wird, wobei n eine ganze Zahl von 1 bis 200 ist.

8. Vorrichtung nach einem oder mehreren der Ansprüche. 1 bis 7, wobei die isotherme Reaktionsstrecke ein thermisch isoliertes Rohr ist.

9. Vorrichtung nach einem oder mehreren der Ansprüche 1 bis 8, worin die isotherme Reaktionsstrecke eine Vorrichtung zur Zu- oder Abfuhr von Energie umfasst.

10. Verfahren zum kontinuierlichen Durchführen chemischer Reaktionen, bei dem das Reaktionsgut in einer Vorrichtung nach einem oder mehreren der Ansprüche 1 bis 9 durch eine Heizzone geführt wird, in der es mittels Mikrowellen auf Reaktionstemperatur erhitzt wird und aus der das erhitzte und gegebenenfalls unter Druck stehende Reaktionsgut direkt nach dem Verlassen der Heizzone in eine sich an die Heizzone anschließende isotherme Reaktionsstrecke überführt und nach Verlassen der isothermen Reaktionsstrecke abgekühlt wird.

11. Verfahren nach Anspruch 10, worin die chemischen Reaktionen durch eine exotherme Wärmetönung von weniger als -20 kJ/mol gekennzeichnet sind.

12. Verfahren nach einem oder mehreren der Ansprüche 10 und 11, worin die chemischen Reaktionen endotherm sind.

13. Verfahren nach einem oder mehreren der Ansprüche 10 bis 12, bei dem das Reaktionsgut durch die Mikrowellenbestrahlung auf Temperaturen zwischen 70 und 500 °C erhitzt wird.

14. Verfahren nach einem oder mehreren der Ansprüche 10 bis 13, bei dem die Mikrowellenbestrahlung bei Drücken oberhalb des Atmosphärendrucks erfolgt.

15. Verfahren nach einem oder mehreren der Ansprüche 10 bis 14, worin die isotherme Reaktionsstrecke so beschaffen ist, dass die Temperatur des Reaktionsgutes nach Durchlaufen der Reaktionsstrecke gegenüber der Eintrittstemperatur um höchstens ± 20 °C abweicht.

16. Verfahren nach einem oder mehreren der Ansprüche 10 bis 15, bei dem sich im Hohlraumresonator eine stehende Welle ausbildet.

## Claims

1. An apparatus for continuously performing chemical reactions, comprising a microwave generator, a microwave applicator with a microwave-transparent tube within, and an isothermal reaction zone, which are arranged such that the reaction mixture in the microwave-transparent tube, the longitudinal axis of which is in the direction of propagation of the microwave, is conducted through a monomode microwave applicator which functions as a heating zone, within which it is heated to reaction temperature by means of microwaves which are conducted out of the microwave generator into the microwave applicator, and within which the heated and optionally pressurized reaction mixture is transferred immediately after leaving the heating zone into an isothermal reaction zone adjoining the heating zone and, after leaving the isothermal reaction zone, is cooled.

2. The apparatus as claimed in claim 1, in which the microwave-transparent tube is within a hollow conductor connected to a microwave generator via waveguides.

3. The apparatus as claimed in claim 1 or 2, in which the microwave applicator is configured as a cavity resonator.

4. The apparatus as claimed in one or more of claims 1 to 3, in which the microwave applicator is configured as a reflection-type cavity resonator.

5. The apparatus as claimed in one or more of claims 1 to 4, in which the microwave-transparent tube is aligned axially with a central axis of symmetry of the hollow conductor.

6. The apparatus as claimed in one or more of claims 1 to 5, in which the cavity resonator has a coaxial transition of the microwaves.

7. The apparatus as claimed in one or more of claims 1 to 6, in which the cavity resonator is operated in E₀₁ₙ mode where n is an integer from 1 to 200.

8. The apparatus as claimed in one or more of claims 1 to 7, wherein the isothermal reaction zone is a thermally insulated tube.

9. The apparatus as claimed in one or more of claims 1 to 8, in which the isothermal reaction zone comprises an apparatus for supply or removal of energy.

10. A process for continuously performing chemical reactions, in which the reaction mixture, in an apparatus as claimed in one or more of claims 1 to 9, is conducted through a heating zone in which it is heated to reaction temperature by means of microwaves and from which the heated and optionally pressurized reaction mixture is transferred immediately after leaving the heating zone into an isothermal reaction zone adjoining the heating zone and, after leaving the isothermal reaction zone, is cooled.

11. The process as claimed in claim 10, in which the chemical reactions are **characterized by** an exothermicity of less than -20 kJ/mol.

12. The process as claimed in one or more of claims 10 and 11, in which the chemical reactions are endothermic.

13. The process as claimed in one or more of claims 10 to 12, in which the reaction mixture is heated by the microwave irradiation to temperatures between 70 and 500°C.

14. The process as claimed in one or more of claims 10 to 13, in which the microwave irradiation is effected at pressures above atmospheric pressure.

15. The process as claimed in one or more of claims 10 to 14, in which the properties of the isothermal reaction zone are such that the temperature of the reaction mixture after passing through the reaction zone differs by at most ± 20°C from the inlet temperature.

16. The process as claimed in one or more of claims 10 to 15, in which a standing wave forms in the cavity resonator.

## Revendications

1. Dispositif pour la réalisation continue de réactions chimiques, comprenant un générateur de micro-ondes, un applicateur de micro-ondes dans lequel se trouve un tube transparent aux micro-ondes, et une section de réaction isotherme, qui sont agencés de manière à ce que le mélange réactionnel dans le tube transparent aux micro-ondes, dont l'axe longitudinal se trouve dans la direction de propagation des micro-ondes, traverse un applicateur de micro-ondes monomodal fonctionnant en tant que zone de chauffage, dans lequel il est porté à une température de réaction au moyen de micro-ondes conduites à partir du générateur de micro-ondes dans l'applicateur de micro-ondes, et dans lequel le mélange réactionnel chauffé et éventuellement sous pression est transféré directement à la sortie de la zone de chauffage dans une section de réaction isotherme suivant la zone de chauffage et refroidi à la sortie de la section de réaction isotherme.

2. Dispositif selon la revendication 1, dans lequel le tube transparent aux micro-ondes se trouve dans un conducteur creux raccordé avec un générateur de micro-ondes par un guide d'ondes.

3. Dispositif selon la revendication 1 ou 2, dans lequel l'applicateur de micro-ondes est configuré sous la forme d'une cavité résonante.

4. Dispositif selon une ou plusieurs des revendications 1 à 3, dans lequel l'applicateur de micro-ondes est configuré sous la forme d'une cavité résonante de type à réflexion.

5. Dispositif selon une ou plusieurs des revendications 1 à 4, dans lequel le tube transparent aux micro-ondes est aligné axialement avec un axe de symétrie central du conducteur creux.

6. Dispositif selon une ou plusieurs des revendications 1 à 5, dans lequel la cavité résonante présente un croisement coaxial des micro-ondes.

7. Dispositif selon une ou plusieurs des revendications 1 à 6, dans lequel la cavité résonante est utilisée en mode E₀₁ₙ, n étant un nombre entier de 1 à 200.

8. Dispositif selon une ou plusieurs des revendications 1 à 7, dans lequel la section de réaction isotherme est un tube isolé thermiquement.

9. Dispositif selon une ou plusieurs des revendications 1 à 8, dans lequel la section de réaction isotherme comprend un dispositif d'apport ou de dissipation d'énergie.

10. Procédé de réalisation continue de réactions chimiques, dans lequel le mélange réactionnel traverse une zone de chauffage dans un dispositif selon une ou plusieurs des revendications 1 à 9, dans laquelle il est porté à une température de réaction au moyen de micro-ondes et à partir de laquelle le mélange réactionnel chauffé et éventuellement sous pression est transféré directement à la sortie de la zone de chauffage dans une section de réaction isotherme suivant la zone de chauffage et refroidi à la sortie de la section de réaction isotherme.

11. Procédé selon la revendication 10, dans lequel les réactions chimiques sont **caractérisées par** une chaleur de réaction exothermique inférieure à -20 kJ/mol.

12. Procédé selon une ou plusieurs des revendications 10 et 11, dans lequel les réactions chimiques sont endothermes.

13. Procédé selon une ou plusieurs des revendications 10 à 12, dans lequel le mélange réactionnel est porté à des températures comprises entre 70 et 500 °C par le rayonnement micro-onde.

14. Procédé selon une ou plusieurs des revendications 10 à 13, dans lequel l'exposition aux micro-ondes a lieu à des pressions supérieures à la pression atmosphérique.

15. Procédé selon une ou plusieurs des revendications 10 à 14, dans lequel la section de réaction isotherme est conçue de manière à ce que la température du mélange réactionnel après la traversée de la section de réaction diffère d'au plus ± 20 °C de la température d'entrée.

16. Procédé selon une ou plusieurs des revendications 10 à 15, dans lequel une onde stationnaire se forme dans la cavité résonante.
